# EUROPEAN PATENT APPLICATION

(11) **EP 3 050 944 A1**
(43) Date of publication of application: **03.08.2016**
(21) Application number: 16151772.7
(22) Date of filing: 19.01.2016
(51) Int. Cl.: C10L 3/10, C10G 25/00, B01J 27/043, B01J 35/00, B01J 37/20, B01J 23/80, B01J 27/045, B01J 35/02

(54) **DESULFURIZATION PROCESS AND A DESULFURIZER**

(30) Priority: 02.02.2015 JP 2015018678
(71) Applicant: Panasonic Intellectual Property Management Co., Ltd., Osaka-shi, Osaka 540-6207 (JP)
(72) Inventor: WAKITA, Hidenobu, Osaka-shi, Osaka 540-6207 (JP); SATOKAWA, Shigeo, Osaka-shi, Osaka 540-6207 (JP)
(74) Representative: Grünecker Patent- und Rechtsanwälte PartG mbB

(57) **Abstract**

A desulfurization process includes desulfurizing a hydrocarbon fuel using a desulfurizing agent including a support, nickel sulfide on the support, and zinc oxide. The desulfurizing agent is heated to 250°C or more.

## Description

### BACKGROUND

### 1. Technical Field

The present disclosure relates to a desulfurization process and a desulfurizer.

### 2. Description of the Related Art

Offering high power-generation and overall efficiency, fuel cell cogeneration systems (hereinafter simply referred to as "fuel cell systems") have been receiving attention as generators for distributed generation, a form of power generation capable of effective use of energy. Many of fuel cells, e.g., phosphoric acid fuel cells and solid polymer fuel cells, use hydrogen as a fuel for power generation. Usually, however, hydrogen supply which is needed when such a fuel cell is used to generate electricity is not available as infrastructure, and hydrogen needs to be produced at the place where the fuel cell system is installed. For this reason, known fuel cell systems often include a hydrogen generator in addition to a fuel cell. The hydrogen generator produces hydrogen from a hydrocarbon fuel such as natural gas or LPG using, for example, steam reforming, a process for producing hydrogen.

Natural gas usually contains several ppm of a sulfur compound such as tertiary butyl mercaptan (TBM), dimethyl sulfide (DMS), or tetrahydrothiophene (THT) as an odorant, besides sulfur compounds derived from fuel. Catalysts for steam reforming are, in general, likely to be poisoned by such a sulfur compound. Hence steam reforming catalysts are used with a desulfurizer as a pretreatment. There are two well-known processes for desulfurization: adsorption desulfurization, which involves desulfurization through adsorption at the ambient temperature and regular replacement of the desulfurizer; and hydrodesulfurization, in which a reforming gas discharged at 200°C to 400°C from a hydrogen generator is recycled into the desulfurizer, the hydrogen in the recycled gas is attached to sulfur using a CoMo catalyst or similar, and the resulting hydrogen sulfide is removed using zinc oxide or similar. Another known example is ultra-deep desulfurization, a process in which a CuZnO-based desulfurizing agent is used to hydrogenate sulfur compounds at 200°C to 350°C and, at the same time, absorbs sulfur (for example, see Japanese Unexamined Patent Application Publication No. 6-256779).

### SUMMARY

Adsorption desulfurization involves absorbing sulfur compounds at the ambient temperature using zeolite containing a transition metal such as Ag, Cu, or Mn, but the adsorption capacity of the zeolite for sulfur compounds is decreased by the water existing in the gas to be desulfurized. Some researchers have proposed Ag-zeolite, arguing that it can be used substantially regardless of the dew point of the gas and is capable of effectively removing dimethyl sulfide (DMS), a hardly adsorbable sulfur compound (for example, see S. Satokawa, Y. Kobayashi, H. Fujiki, Applied Catalysis B: Environmental 56 (2005) 51). This material, however, has some disadvantages such as the high cost of using Ag.

As a solution to this problem, desulfurizing agents have been reported that are used at high temperatures (150°C to 300°C), at which water in the fuel gas is less inhibitory (for example, see Japanese Unexamined Patent Application Publication No. 2009-134890). These desulfurizing agents only insufficiently remove the hardly adsorbable DMS and thus need to be used with a large amount of adsorbent, causing problems such as increased heat capacity. For example, desulfurizing agents such as those incorporating nickel oxide have been proposed (for example, see Japanese Unexamined Patent Application Publication No. 11-253742), but these do not perform sufficiently in removing DMS.

On the other hand, hydrodesulfurization, which involves recycling part of a hydrogen-containing reforming gas to feed it to a hydrocarbon fuel while maintaining the temperature of the desulfurizing agent at 200°C to 350°C, has disadvantages such as the complicated structure of the system.

One non-limiting and exemplary embodiment provides a desulfurization process and a desulfurizer that both enable the user to remove sulfur compounds from a hydrocarbon fuel effectively and for a long period of time more easily than ever, without hydrodesulfurization of the hydrocarbon fuel.

After extensive research to solve the aforementioned problems, the inventors found that the use of a particular desulfurizing agent, rather than hydrodesulfurization of the hydrocarbon fuel, leads to long-term stable removal of sulfur compounds existing in the hydrocarbon fuel, in particular, the hardly adsorbable DMS, and completed an aspect of the present disclosure.

In one general aspect, the techniques disclosed here feature a desulfurization process. The desulfurization process involves desulfurizing a hydrocarbon fuel using a desulfurizing agent including a support, nickel sulfide on the support, and zinc oxide. The desulfurizing agent is heated to 250°C or more.

The desulfurization process and the desulfurizer according to an aspect of the present disclosure enable the user to remove sulfur compounds from a hydrocarbon fuel effectively and for a long period of time more easily than ever, without hydrodesulfurization of the hydrocarbon fuel.

It should be noted that general or specific embodiments may be implemented as a system, a method, an integrated circuit, a computer program, a storage medium, or any selective combination thereof.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### BRIEF DESCRIPTION OF THE DRAWINGS

Fig. 1 illustrates an example of a procedure for preparing a nickel oxide catalyst;
Fig. 2 illustrates an example of a study on the desulfurization ability of a nickel oxide catalyst;
Fig. 3 illustrates an example of a study on the desulfurization ability of a nickel sulfide catalyst;
Fig. 4A illustrates an example of a study on the desulfurization ability of a zinc oxide catalyst with DMS;
Fig. 4B illustrates an example of a study on the desulfurization ability of a zinc oxide catalyst with methyl mercaptan;
Fig. 5A illustrates an example of a desulfurizer according to an example of an embodiment;
Fig. 5B illustrates an example of a desulfurizer according to an example of an embodiment;
Fig. 5C illustrates an example of a desulfurizer according to an example of an embodiment;
Fig. 5D illustrates an example of a desulfurizer according to a comparative example;
Fig. 6 illustrates an example of a study on the desulfurization ability of the desulfurizer illustrated in Fig. 5A;
Fig. 7 illustrates an example of a study on the desulfurization ability of the desulfurizer illustrated in Fig. 5B;
Fig. 8 illustrates an example of a study on the desulfurization ability of the desulfurizer illustrated in Fig. 5C; and
Fig. 9 illustrates an example of a study on the desulfurization ability of the desulfurizer illustrated in Fig. 5D.

### DETAILED DESCRIPTION

Through extensive research on various known problems with the removal of sulfur compounds from hydrocarbon fuels, the inventors found the following.

Hydrocarbon fuels such as manufactured gas and LP gas contain a trace amount of a sulfur compound-based odorant which is intentionally mixed therein to serve as an indicator for gas leakage. Different gas suppliers use different odorants, but in Japan, TBM, DMS, and THT are usually used, in concentrations of several ppm. Unlike natural gas and coke oven gas, which contain a wide range of sulfur compounds, manufactured gas contains a sulfur compound with a known structure intentionally mixed therein. Of these sulfur compounds, DMS is abundant and difficult to remove. The research by the inventors thus focused on the removal of DMS.

As mentioned above, desulfurizing agents incorporating nickel oxide have been reported but do not perform well in removing DMS. However, the inventors found that the ability of nickel oxide to remove DMS improves over time when the desulfurization reaction is continued. This finding indicated that when turned into a sulfide and then used to remove DMS, nickel oxide can perform excellently in removing DMS from early in its use, thereby leading the inventors to the development of such a desulfurizing agent. To be more specific, heating sulfurized nickel (nickel sulfide) to a high temperature (250°C or more) makes the compound a superb DMS remover that performs excellently from early in its use.

Decomposition and removal of DMS using nickel sulfide, however, can produce methyl mercaptan, a sulfur-containing component, or similar by-products. The inventors thus investigated various methyl mercaptan removers, with the finding that zinc oxide is capable of removing methyl mercaptan but this process generates DMS as a by-product. When nickel sulfide and zinc oxide are used in combination, therefore, DMS can be removed without emission of methyl mercaptan or similar by-products. Desulfurizing agents only incorporating zinc oxide do not remove DMS at temperature of 350°C or less.

Furthermore, zinc oxide exhibits a high adsorption capacity for methyl mercaptan and hydrogen sulfide at 350°C. There are also other materials capable of adsorbing methyl mercaptan and hydrogen sulfide, including manganese oxide, cerium oxide, lanthanum oxide, zirconium oxide, and titanium oxide, metal oxides that carry noble metals such as Rh, Pd, Ru, Pt, and Ag, and Ni-carrying metal oxides.

The desulfurization process according to a first aspect of the present disclosure is therefore a desulfurization process that involves desulfurizing a hydrocarbon fuel using a desulfurizing agent that includes a support, nickel sulfide on the support, and zinc oxide, wherein the desulfurizing agent is heated to 250°C or more. The desulfurizer according to a first aspect of the present disclosure is a desulfurizer including a desulfurizing agent to remove a sulfur compound from a hydrocarbon fuel, the desulfurizing agent including a support, nickel sulfide on the support, and zinc oxide, wherein the desulfurizing agent has been heated to 250°C or more.

With these, the user can remove sulfur compounds from a hydrocarbon fuel effectively and for a long period of time more easily than ever, without hydrodesulfurization of the hydrocarbon fuel.

In the desulfurization process according to a second aspect of the present disclosure, the support in the desulfurization process according to the first aspect may be alumina.

Alumina is an inexpensive material and, furthermore, has a large specific surface area. The use of alumina as the support therefore ensures a high degree of dispersion of the nickel on the alumina, leading to an increased number of active sites.

In the desulfurization process according to a third aspect of the present disclosure, the nickel sulfide in the desulfurization process according to the first aspect may be prepared through sulfurization of nickel oxide held on the support.

In the desulfurization process according to a fourth aspect of the present disclosure, it is better that in the desulfurization process according to the first aspect, the desulfurizing agent be heated to 250°C or more and 450°C or less.

If the temperature of the desulfurizing agent exceeds 450°C, the deposition of carbon derived from decomposing sulfur compounds is remarkable. This situation often leads to breaks in (i.e., the destruction of) the desulfurizing agent which can cause increased pressure drop. The risk for this can be reduced by controlling the temperature of the desulfurizing agent as specified above.

In the desulfurizer according to a fifth aspect of the present disclosure, it is better that the desulfurizer according to the first aspect further include a reactor and first and second catalyst layers in the reactor, the first and second catalyst layers containing a predetermined size of the nickel sulfide catalyst particles and a predetermined size of the zinc oxide particles, respectively, and arranged in this order from upstream in the direction of flow of the hydrocarbon fuel.

In the desulfurizer according to a sixth aspect of the present disclosure, it is better that the desulfurizer according to the first aspect further include a reactor mix-loaded with a predetermined size of the nickel sulfide catalyst particles and a predetermined size of the zinc oxide particles.

With these, the user can remove sulfur compounds at a lower temperature than in the case where a mixture of nickel sulfide catalyst and zinc oxide is granulated into a predetermined size of catalyst particles in which the two compounds coexist in each particle.

### Embodiments

The following describes an embodiment of the present disclosure in detail with reference to drawings. In the drawings, identical or equivalent components are denoted by the same reference numerals so that duplicate descriptions are omitted.

### Preparation of a nickel oxide catalyst

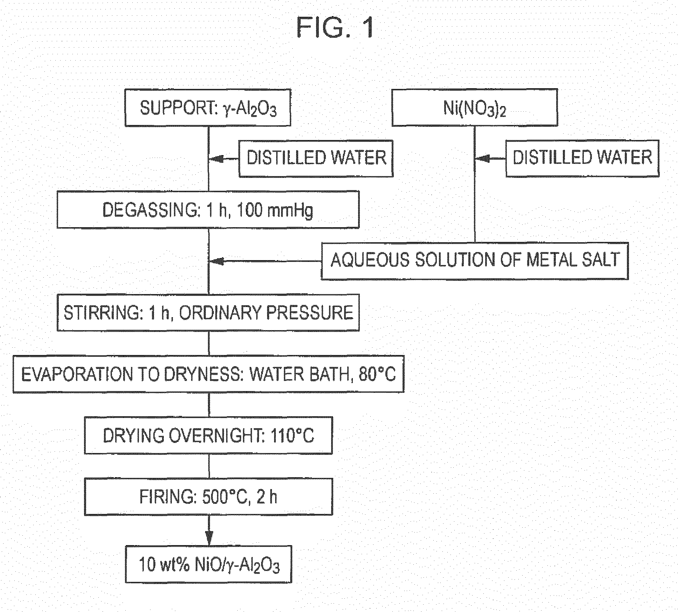

Fig. 1 illustrates an example of a procedure for preparing a nickel oxide catalyst.

First, "Catapal^{®} B" alumina (Sasol) was fired at 600°C for 2 hours. Distilled water was added to the obtained γ-Al₂O₃, and the resulting mixture was degassed at 100 mmHg for 1 hours.

The degassed mixture was then stirred with an aqueous solution of nickel nitrate (Wako Pure Chemical Industries) for 1 hour.

The mixture was then evaporated at 200 mmHg and 80°C for 2 hours to dryness. The residue was dried at 110°C overnight and then fired at 500°C for 2 hours.

In this way, a NiO/γ-Al₂O₃ catalyst containing the equivalent of 10% by weight metallic nickel (hereinafter referred to as "the 10 wt% NiO/γ-Al₂O₃ catalyst") was prepared.

A fine powder of the 10 wt% NiO/γ-Al₂O₃ catalyst was then granulated into particles having an average particle size of approximately 200 µm (not illustrated). Instead of compaction, it is also possible to control the size of the catalyst particles using an appropriate inorganic binder (e.g., silica or any similar inorganic binder).

### Study on the desulfurization ability of the nickel oxide catalyst

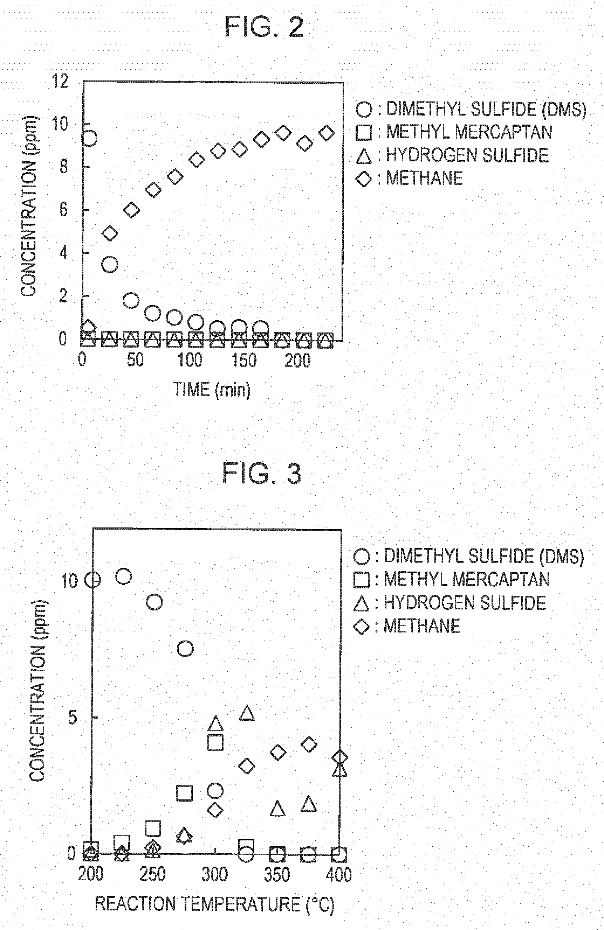

Fig. 2 illustrates an example of a study on the desulfurization ability of the nickel oxide catalyst. The plots in Fig. 2 represent the relationships between time (minutes), on the horizontal axis, and the concentrations (ppm) of some components of a gas passed through the 10 wt% NiO/γ-Al₂O₃ catalyst, on the vertical axis.

In this study, 500 mg of the 10 wt% NiO/γ-Al₂O₃ catalyst was loaded in a fixed-bed flow reactor. Then a N₂ gas containing 10 ppm DMS was passed through the catalyst maintained at 350°C, at a flow rate of 500 mL/min (a GHSV of 40000 h⁻¹). The gas discharged from the outlet of the fixed-bed flow reactor was analyzed using gas chromatography (with an FPD) for changes in the concentrations of some components of the gas over time.

As can be seen from Fig. 2, the concentration of DMS suddenly dropped at approximately 25 minutes after the start of the study, and was below the limit of detection from approximately 150 minutes onward. The concentration of methane increased from the initial undetectable level, suggesting that sulfurization of the 10 wt% NiO/γ-Al₂O₃ catalyst improved the activity of this catalyst in decomposing DMS.

Thus, the inventors prepared a nickel sulfide catalyst by sulfurizing the 10 wt% NiO/γ-Al₂O₃ catalyst as follows to further investigate the activity of such a catalyst in decomposing DMS.

### Preparation of a nickel sulfide catalyst

With the 10 wt% NiO/γ-Al₂O₃ catalyst obtained in the preparation procedure illustrated in Fig. 1 maintained at 500°C, a N₂ gas containing 1000 ppm H₂S was passed through it at a flow rate of 200 mL/min for sulfurization in such a manner that the hydrogen sulfide flowed at least in an amount stoichiometrically equivalent to the nickel held on the support. In this way, a 10 wt% NiS/γ-Al₂O₃ catalyst was prepared as a catalyst stabilized in terms of performance.

### Study on the desulfurization ability of the nickel sulfide catalyst

Fig. 3 illustrates an example of a study on the desulfurization ability of the nickel sulfide catalyst. The plots in Fig. 3 represent the relationships between reaction temperature, on the horizontal axis, and the concentrations (ppm) of some components of a gas passed through the 10 wt% NIS/γ-Al₂O₃ catalyst, on the vertical axis.

In this study, the catalytic activity of the 10 wt% NiS/γ-Al₂O₃ catalyst was investigated in the temperature range of 200°C to 500°C using the same reactor and under the same conditions as in the study illustrated in Fig. 2.

As can be seen from Fig. 3, the concentration of DMS decreased with increasing reaction temperature and fell below the limit of detection at 325°C. The concentration of methyl mercaptan, a sulfur-containing component and by-product of the decomposition of DMS, increased at temperatures from 225°C to 325°C, and the concentration of methane, another by-product, increased at 275°C and higher temperatures. Hydrogen sulfide also formed at 275°C and higher temperatures, suggesting that the 10 wt% NiS/γ-Al₂O₃ catalyst drove the decomposition of DMS.

Although in this example N₂ gas was used as balance gas instead of a hydrocarbon fuel, the same advantages are available with a hydrocarbon fuel. Examples of the hydrocarbon fuel include, besides manufactured gas and natural gas, ethane, propane, LPG (liquid petroleum gas), naphtha, gas oil, and kerosene.

After the DMS-decomposing reactions performed in the temperature range of 350°C to 500°C, the 10 wt% NiS/γ-Al₂O₃ catalyst was analyzed for carbon and sulfur. The analysis revealed that the quantities of carbon and sulfur on the 10 wt% NiS/γ-Al₂O₃ catalyst both increased with elevating reaction temperature. In light of the fact that remarkable deposition of carbon causes the destruction of the 10 wt% NiS/γ-Al₂O₃ catalyst and leads to increased pressure drop, it is better that the 10 wt% NiS/γ-Al₂O₃ catalyst be thermally controlled (heated) to a temperature that allows DMS to decompose but is not so high that the deposition of carbon is remarkable. To be more specific, it is preferred that the temperature of the 10 wt% NiS/γ-Al₂O₃ catalyst be 250°C or more and 450°C or less, more preferably 275°C or more and 350°C or less.

### Study on the desulfurization ability of a zinc oxide catalyst

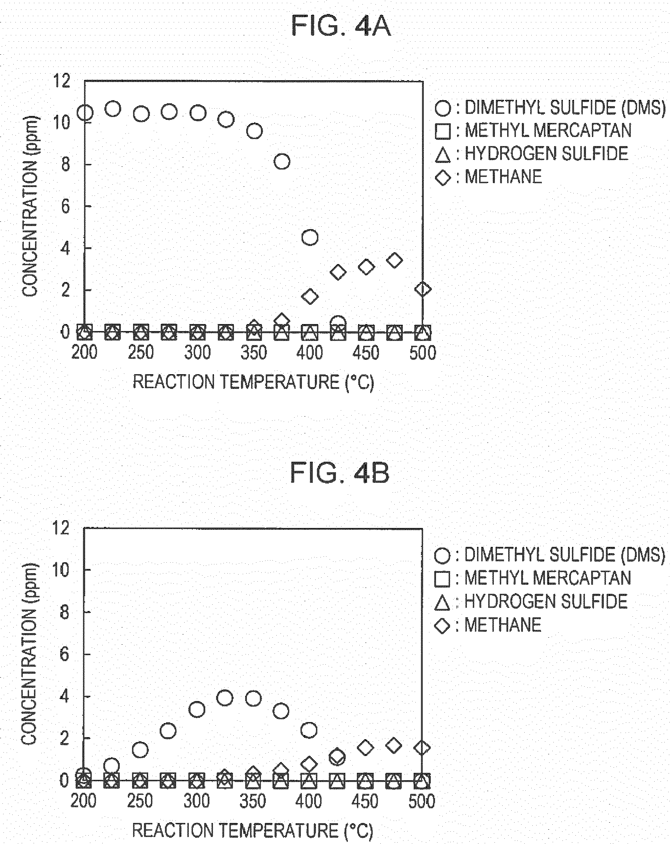

Fig. 4A illustrates an example of a study on the desulfurization ability of a zinc oxide catalyst with DMS. Fig. 4B illustrates an example of a study on the desulfurization ability of the zinc oxide catalyst with methyl mercaptan. The plots in Figs. 4A and 4B represent the relationships between reaction temperature, on the horizontal axis, and the concentrations (ppm) of some components of gases passed through the ZnO catalyst, on the vertical axis.

In the first study, the temperature dependence of the decomposition of DMS with "ActiSorb^{®} S2" zinc oxide (Clariant) as a ZnO catalyst was investigated in the temperature range of 200°C to 500°C using the same reactor and under the same conditions as in the study illustrated in Fig. 2.

As can be seen from Fig. 4A, DMS decomposed at 350°C and higher temperatures and decreased to a concentration below the limit of detection at 450°C. No methyl mercaptan was detected between 200°C and 500°C.

Then a gas containing 10 ppm methyl mercaptan instead of DMS was passed through the zinc oxide. As is presented in Fig. 4B, DMS was emitted between 200°C and 450°C.

These results suggest that ZnO catalysts are less effective in decomposing DMS than the 10 wt% NiS/γ-Al₂O₃ catalyst and that removing methyl mercaptan on a ZnO catalyst generates DMS as a by-product.

In this embodiment, therefore, the inventors used a desulfurizing agent including a support such as alumina, nickel sulfide on the support, and zinc oxide, and found that with such a desulfurizing agent, it is possible to remove DMS from a hydrocarbon fuel effectively and for a long period of time more easily than ever, without hydrodesulfurization of the hydrocarbon fuel. To be more specific, the 10 wt% NiS/γ-Al₂O₃ catalyst, when heated to a high temperature (250°C or more), serves as an excellent DMS remover from early in its use. Furthermore, the ZnO catalyst, coexisting with the 10 wt% NiS/γ-Al₂O₃ catalyst, reduces the emission of methyl mercaptan or similar by-products to the outlet associated with the decomposition of DMS by the 10 wt% NiS/γ-Al₂O₃ catalyst.

There are also other materials capable of adsorbing methyl mercaptan and hydrogen sulfide, including manganese oxide, cerium oxide, lanthanum oxide, zirconium oxide, and titanium oxide, metal oxides that carry noble metals such as Rh, Pd, Ru, Pt, and Ag, and Ni-carrying metal oxides, and such materials can also be used in combination with the NiS/γ-Al₂O₃ catalyst. In terms of adsorption capacity, zinc oxide is the best.

### Examples

The following describes some desulfurizers as examples of the above embodiment with reference to drawings.

### Structure of devices

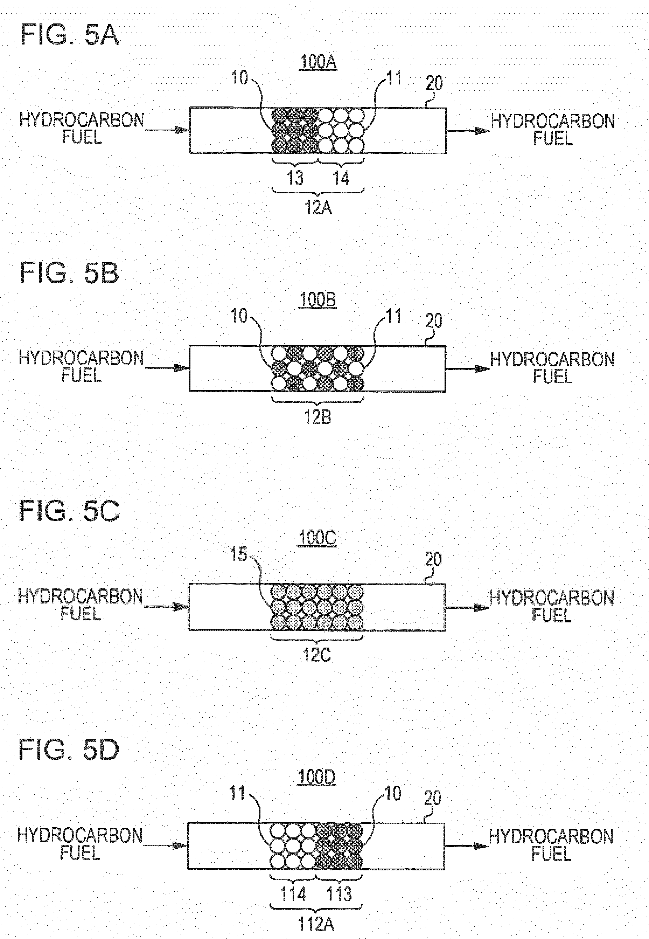

Figs. 5A, 5B, and 5C illustrate examples of desulfurizers according to examples of the embodiment. Fig. 5D illustrates an example of a desulfurizer according to a comparative example.

The desulfurizer 100A illustrated in Fig. 5A includes a desulfurizing agent 12A to remove a sulfur compound such as DMS from a hydrocarbon fuel. The desulfurizing agent 12A includes a support such as alumina, nickel sulfide on the support, and zinc oxide and has been heated to 250°C or more with an appropriate heater (not illustrated). In this example, each of 10 wt% NiS/γ-Al₂O₃ and ZnO has been granulated into particles having a predetermined size (e.g., an average particle size of approximately 200 µm), and there are in a reactor 20 a first catalyst layer 13 containing 10 wt% NiS/γ-Al₂O₃ catalyst particles 10 of this size and a second catalyst layer 14 containing ZnO catalyst particles 11 of the same size, arranged in this order from upstream in the direction of flow of the hydrocarbon fuel. To be more specific, a fine powder of 10 wt% NiS/γ-Al₂O₃ and a fine powder of ZnO have each been made into catalyst particles 10 and catalyst particles 11, respectively, having an average particle size of 200 µm, and the reactor 20 contains 250 mg each of the two groups of catalyst particles, with the catalyst particles 10 upstream and the catalyst particles 11 downstream (hereinafter referred to as "bilayer loading").

The desulfurizer 100B illustrated in Fig. 5B includes a desulfurizing agent 12B to remove a sulfur compound such as DMS from a hydrocarbon fuel. The desulfurizing agent 12B includes a support such as alumina, nickel sulfide on the support, and zinc oxide and has been heated to 250°C or more with an appropriate heater (not illustrated). In this example, each of 10 wt% NiS/γ-Al₂O₃ and ZnO has been granulated into particles having a predetermined size (e.g., an average particle size of approximately 200 µm), and there are in a reactor 20 a mixture of 10 wt% NiS/γ-Al₂O₃ catalyst particles 10 and ZnO catalyst particles 11 both of this size. To be more specific, a fine powder of 10 wt% NiS/γ-Al₂O₃ and a fine powder of ZnO have each been granulated into catalyst particles 10 and catalyst particles 11, respectively, having an average particle size of 200 µm, and the reactor 20 contains the catalyst particles 10 and the catalyst particles 11 in such a manner that 250 mg each of the two groups of catalyst particles are uniformly mixed in the reactor 20 (hereinafter referred to as "mix-loading").

The desulfurizer 100C illustrated in Fig. 5C includes a desulfurizing agent 12C to remove a sulfur compound such as DMS from a hydrocarbon fuel. The desulfurizing agent 12C includes a support such as alumina, nickel sulfide on the support, and zinc oxide and has been heated to 250"C or more with an appropriate heater (not illustrated). In this example, a mortar-ground 1:1 (w/w) mixture of a fine powder of 10 wt% NiS/γ-Al₂O₃ and a fine powder of ZnO has been granulated into catalyst particles 15 having an average particle size of 200 µm, and the reactor 20 contains a total of 500 mg of the catalyst particles 15 containing 10 wt% NiS/γ-Al₂O₃ and ZnO (hereinafter referred to as "physical mixing").

The desulfurizer 100D illustrated in Fig. 5D includes a desulfurizing agent 112A as a comparative example of bilayer loading. There are a first catalyst layer 114 containing ZnO catalyst particles 11 upstream in a reactor 20 and a second catalyst layer 113 containing 10 wt% NiS/γ-Al₂O₃ catalyst particles 10 downstream in the reactor 20.

### Studies on the desulfurization ability of the desulfurizers

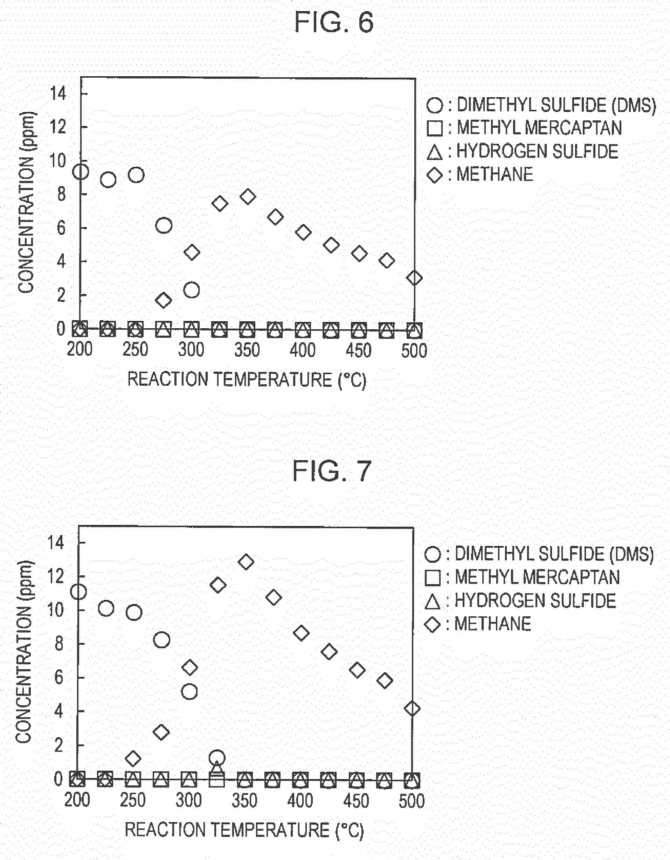

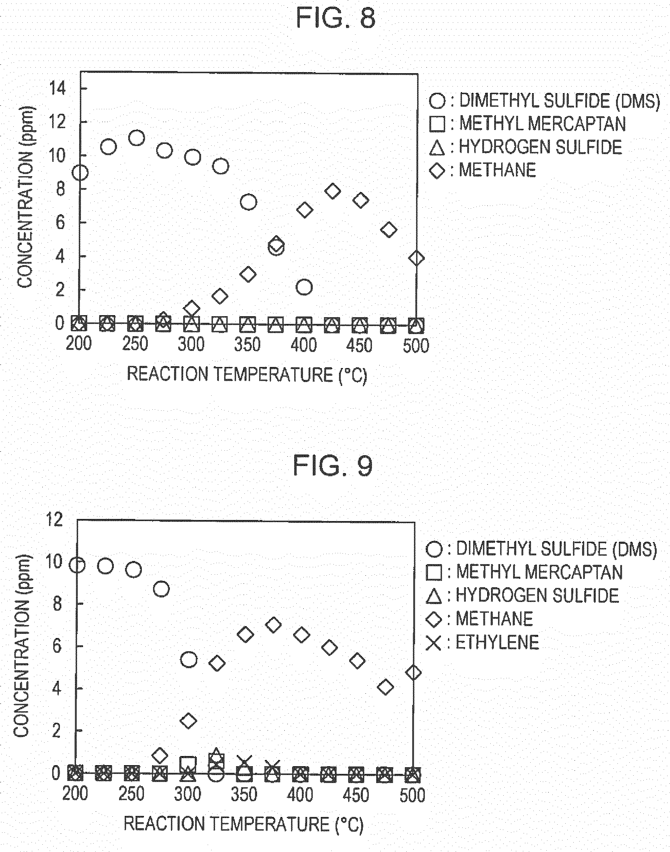

Fig. 6 illustrates an example of a study on the desulfurization ability of the desulfurizer illustrated in Fig. 5A. Fig. 7 illustrates an example of a study on the desulfurization ability of the desulfurizer illustrated in Fig. 5B. Fig. 8 illustrates an example of a study on the desulfurization ability of the desulfurizer illustrated in Fig. 5C. Fig. 9 illustrates an example of a study on the desulfurization ability of the desulfurizer illustrated in Fig. 5D. The plots in each of Figs. 6, 7, 8, and 9 represent the relationships between reaction temperature, on the horizontal axis, and the concentrations (ppm) of some components of gases passed through the desulfurizer 100A, 100B, 100C, or 100D, on the vertical axis.

In these studies, the desulfurization ability of the desulfurizers 100A, 100B, 100C, and 100D was investigated in the temperature range of 200°C to 500°C using the same reactor and under the same conditions as in the study illustrated in Fig. 2.

As is presented in Figs. 6, 7, and 8, for all of the bilayer-loading desulfurizer 100A, the mix-loading desulfurizer 100B, and the physical-mixing desulfurizer 100C, the concentration of hydrogen sulfide was 1 ppm or less across the temperature range studied, and methyl mercaptan was also less than the lower limit of detection across the entire temperature range.

The decomposition of DMS proceeded at 250°C and higher temperatures in all of these desulfurizers. When using any of the desulfurizers 100A, 100B, and 100C, therefore, it is preferred to heat the desulfurizing agent 12A, 12B, or 12C to 250°C or more. As mentioned above, it is better that the temperature of the desulfurizing agent 12A, 12B, or 12C be controlled (or the desulfurizing agent be heated) to a temperature not so high that the deposition of carbon is remarkable. To be more specific, it is preferred that the temperature of the desulfurizing agent 12A, 12B, or 12C be 250°C or more and 450°C or less. For the desulfurizing agents 12A and 12B, it is more preferred that the temperature be 275°C or more and 350°C or less.

The temperature at which DMS was completely decomposed was approximately 425°C for the physical-mixing desulfurizer 100C, approximately 350°C for the mix-loading desulfurizer 100B, and approximately 325°C for the bilayer-loading desulfurizer 100A ("physical mixing" > "mix-loading" > "bilayer loading"). The bilayer-loading desulfurizer 100A and the mix-loading desulfurizer 100B, achieving complete decomposition of DMS at lower temperatures than the physical-mixing desulfurizer 100C, therefore have a better desulfurization ability than the desulfurizer 100C.

The inventors also performed a desulfurization study with DMS on the desulfurizer 100D, which had a first catalyst layer 114 containing ZnO catalyst particles 11 upstream and a second catalyst layer 113 containing 10 wt% NiS/γ-Al₂O₃ catalyst particles 10 downstream. As is presented in Fig. 9, emission of methyl mercaptan was observed between 300°C and 325°C.

The desulfurization process and the desulfurizer according to an aspect of the present disclosure enable the user to remove sulfur compounds from a hydrocarbon fuel effectively and for a long period of time more easily than ever, without hydrodesulfurization of the hydrocarbon fuel. An aspect of the present disclosure can therefore be applied to, for example, desulfurization processes and desulfurizers for fuel cell systems or similar.

## Claims

1. A desulfurization process comprising:
desulfurizing a hydrocarbon fuel using a desulfurizing agent including a support, nickel sulfide on the support, and zinc oxide, wherein
the desulfurizing agent is heated to 250°C or more.

2. The desulfurization process according to Claim 1, wherein the support is alumina.

3. The desulfurization process according to Claim 1, wherein the nickel sulfide is prepared through sulfurization of nickel oxide held on the support.

4. The desulfurization process according to Claim 1, wherein the desulfurizing agent is heated to 250°C or more and 450°C or less.

5. A desulfurizer comprising:
a desulfurizing agent to remove a sulfur compound from a hydrocarbon fuel, the desulfurizing agent including a support, nickel sulfide on the support, and zinc oxide, wherein
the desulfurizing agent has been heated to 250°C or more.

6. The desulfurizer according to Claim 5, further comprising:
a reactor and first and second catalyst layers in the reactor, the first and second catalyst layers containing a predetermined size of the nickel sulfide catalyst particles and a predetermined size of the zinc oxide particles, respectively, and arranged in this order from upstream in the direction of flow of the hydrocarbon fuel.

7. The desulfurizer according to Claim 5, further comprising:
a reactor mix-loaded with a predetermined size of the nickel sulfide catalyst particles and a predetermined size of the zinc oxide particles.
